# EUROPEAN PATENT SPECIFICATION

(11) **EP 4 013 299 B1**
(45) Date of publication and mention of the grant of the patent: **13.05.2026**
(21) Application number: 20852897.6
(22) Date of filing: 14.08.2020
(51) Int. Cl.: A61B 5/021, A61B 5/022, A61B 5/0295, A61B 5/0215, A61B 5/026, A61B 5/0225, A61B 5/00

(54) **BLOOD PRESSURE MEASUREMENT SYSTEMS AND METHODS**
BLUTDRUCKMESSSYSTEME UND -VERFAHREN
SYSTÈMES ET PROCÉDÉS DE MESURE DE LA PRESSION ARTÉRIELLE

(30) Priority: 14.08.2019 US 201962886368 P
(43) Date of publication of application: 22.06.2022
(73) Proprietor: Cardio Ring Technologies, Inc., San Jose CA 95134 (US)
(72) Inventor: SHIH, Wen-Pin, Taipei, 100 (TW); CHIEN, Wei-Ting, Taoyuan City, 32055 (TW); CHEN, Leng-Chun, Hsinchu City, 30080 (TW)
(74) Representative: Lorenz Seidler Gossel Part. mbB
(86) International application number: PCT/US2020/046480
(87) International publication number: WO 2021/030737

(56) References cited:
- US-A1- 2010 160 798
- US-A1- 2010 249 614
- US-A1- 2010 298 665
- US-A1- 2014 128 747
- US-A1- 2016 310 077
- US-A1- 2016 367 156
- US-A1- 2017 055 855
- US-A1- 2017 188 993
- US-A1- 2019 008 399
- US-A1- 2019 059 825
- GESCHE ET AL.: "Continuous blood pressure measurement by using the pulse transit time: comparison to a cuff-based method", EUROPEAN JOURNAL OF APPLIED PHYSIOLOGY, 10 May 2011 (2011-05-10), XP019999107

## Description

### CROSS-REFERENCE TO RELATED APPLICATIONS

This patent application claims the benefit of U.S. provisional applications No. 62/886,368 filed on August 14, 2019.

### FIELD

This patent specification is in the field of a blood pressure measurement method and device, and more specifically relates to a method and a device for reducing errors in measuring blood pressure.

### BACKGROUND OF THE INVENTION

Cardiovascular disease (CVD) accounts for approximately a significant number of deaths on a world-wide basis. CVD includes coronary heart disease, which accounts for the majority of CVD deaths, as well as stroke and heart failure. CVD is closely related to pathogenic factors and lifestyles. In addition to maintaining a healthy lifestyle, frequent monitoring of blood pressure, glucose, and cholesterol also plays an important role in preventing CVD. To satisfy the demand of preventing or control CVD, there have been many portable bioinformation monitoring devices available for users to measure their own heart rate, blood pressure, glucose, and etc.

In this context, the US-publication US 2019/059825 A1 discloses an indirect oscillometric, digital blood pressure monitoring system enabling self-calibration to obtain absolute blood pressure values. Other systems for blood pressure measurement are known from US 2019/008399 A1 and US 2010/249614 A1.

### TECHNICAL PROBLEMS

Arterial blood pressure is most commonly measured via a sphygmomanometer. Conventionally, when monitoring blood pressure of a patient, during each heartbeat the blood pressure varies between systolic and diastolic pressures. Systolic pressure is a peak pressure in the artery that occurs near the end of the cardiac cycle or contraction. Diastolic pressure is minimum pressure in the artery that occurs near the beginning of the cardiac cycle during filling of the heart with blood.

In conventional portable bioinformation monitoring devices that measure blood pressure, a mean blood pressure is usually measured first, and then a systolic and a diastolic pressure are deduced based on statistical relation between systolic, diastolic, and the measured mean blood pressure. However, the relation between systolic, diastolic, and mean blood pressure might be different due to any number of variations in the particular patient. For example, such variations include age, personal physiology, or life environment. Therefore, conventional bioinformation monitoring devices are prone to inevitable measurement errors. In some variations, portable bioinformation monitoring devices combine a conventional blood pressure cuff with a finger-clip sensor. These devices measure systolic pressure by using the method like the conventional way of applying compressive pressure to the artery to cease flow and then removing the pressure. However, this method still produces measurement errors resulting from motion artifact and respiratory variation of the person being tested. Therefore, there remains a need to produce an improved blood pressure measurement reading that reduces errors and increases increasing an accuracy of measuring actual blood pressure.

### SUMMARY OF INVENTION

In view of this, the systems and methods described herein include blood pressure measurement systems that produce an accurate blood pressure measurement. One variation of the system is to directly measure blood pressure with a portable device configuration and also effectively reduce errors subjected to motion artifact and blood pressure fluctuations caused by respiration.

In a first example, the system allows for blood pressure measurement of an individual and comprises a controller coupled to a pressing unit and to a bioinformation measurement device; where the controller is configured to incrementally increase a pressure applied by the pressing unit on a body part of an individual during a first pressing period to compress the body part to affect blood flow in an upstream blood vessel in the body part; where the bioinformation measurement device is configured to produce a first wave signal representative of blood activity from a downstream blood vessel during the first pressing period and transmitting the first wave signal to the controller; where the controller generates an envelope signal using the first wave signal; wherein the controller is configured to establish a second pressing period by determining when the bioinformation measurement device fails to detect the blood activity; where the bioinformation measurement device is configured to produce a second wave signal of the blood vessel by using the bioinformation measurement device during the second pressing period; where the controller determines a first timepoint where a waveform of the second wave signal intersects with a waveform of the envelope signal to establish a systolic pressure value using a first pressure value that the pressing unit exerts on the upstream blood vessel at the first timepoint; and where the controller also determines a second timepoint where the envelope signal has a predetermined amplitude to establish a diastolic pressure value using a second pressure value that the pressing unit exerts on the upstream blood vessel at the second timepoint.

Variations of the systems described herein can include systems with only a controller that are configured to work with separate bioinformation devices and/or pressing units. For example, such a blood pressure measurement system can include a controller designed for use with a bioinformation measurement device and a pressing unit, the system comprising, where the controller is configured to electronically communicate with the pressing unit and the bioinformation measurement device to perform the functions discussed herein.

A variation of the system includes the first wave signal having a plurality of periodic waves and of generating the envelope signal further comprises: calculating an average value of each periodic wave of the first wave signal; obtaining a first modified wave signal by subtracting the average value from the amplitude of each corresponding periodic wave; and obtaining the envelope signal by connecting a plurality of peaks of the first modified wave signal and a plurality of valleys of the first modified wave signal.

In another variation, the first wave signal has a plurality of periodic waves and the controller generates the envelope signal by connecting peaks of each periodic wave and valleys of each periodic wave.

Another variation of the system includes a controller that is further configured to: detect a third wave signal from the blood vessel by using the bioinformation measurement device during a non-pressing period of the wearable pressing unit, where the third wave signal is a continuous wave; output the systolic pressure value as an initial peak value of a peak in the third wave signal; output the diastolic pressure value as an initial valley value of a valley in the third wave signal that is temporally adjacent to the peak in the third wave signal; and calculate a plurality of additional peak values and a plurality of additional valley value of a plurality of remaining peaks and valleys, respectively, in the third wave signal according to the systolic and diastolic pressure value.

Variations of the controller can determine the first timepoint by: obtaining an average line of the second wave signal; obtaining a modified envelope signal by smoothing the envelope signal; and determine the first timepoint as a time when an upper bound of the modified envelope signal intersects with the average line.

In another variation the controller uses the predetermined amplitude of between 50% and 90% of the maximum amplitude of the envelope signal.

The pressing unit disclosed herein can be configured to fit on an arm or a wrist and where the controller is configured to cause the pressing unit to apply pressure during the first and second pressing period, and where the bioinformation measurement device is configured to detect the first wave and the second wave signal from a finger.

The present disclosure also includes method for blood pressure measurement method comprising: detecting a first wave signal from a blood vessel by using a bioinformation measurement device during a first pressing period of a wearable pressing unit, in which the wearable pressing unit exerts pressure on an upstream blood vessel relative to the blood vessel; generating an envelope signal of the first wave signal according to the first wave signal; detecting a second wave signal of the blood vessel by using the said bioinformation measurement device during a second pressing period of the wearable pressing unit; determining a first timepoint where the waveform of the second wave signal intersects with the waveform of the envelope signal; outputting the pressure value that the wearable pressing unit exerts on the upstream blood vessel at the first timepoint as a systolic pressure value; determining a second timepoint where the envelope signal has a predetermined amplitude; and outputting the pressure value that the wearable pressing unit exerts on the upstream blood vessel at the second timepoint as a diastolic pressure value.

Another variation of the blood pressure measurement device comprises a wearable pressing unit; a bioinformation measurement device; and a control unit with signal connecting to the wearable pressing unit and the bioinformation measurement device, where the control unit executes any one of the methods disclosed herein.

The pressing unit disclosed herein can be any type of blood pressure cuff or other device that performs the function of reducing flow in an upstream vessel. In addition, the bioinformation measurement device can be a finger-clip device having a pressure sensor for detecting the first wave signal, the second wave signal and the third wave signal. The finger-clip device can comprise an optical sensor, or any sensor allowing it to detecting wave signals from the downstream vessel.

In one variation the present systems and methods provide a blood pressure measurement for detecting a first wave signal from a blood vessel during a first pressing period of a wearable pressing unit, in which the wearable pressing unit exerts pressure on an upstream blood vessel relative to the blood vessel; generating an envelope signal of the first wave signal according to the first wave signal; detecting a second wave signal of the blood vessel during a second pressing period of the wearable pressing unit; determining a first timepoint where the waveform of the second wave signal intersects with the waveform of the envelope signal; outputting the pressure value that the wearable pressing unit exerts on the upstream blood vessel at the first timepoint as a systolic pressure value; determining a second timepoint where the envelope signal has a predetermined amplitude; and outputting the pressure value that the wearable pressing unit exerts on the upstream blood vessel at the second timepoint as a diastolic pressure value.

Another proposal of the present invention is to provide a blood pressure measurement device, including a wearable pressing unit, a bioinformation measurement device, and a control unit. The signal of the control unit connects to the wearable pressing unit and the bioinformation measurement device in order to execute the above-mentioned blood pressure measurement method.

### BRIEF DESCRIPTION OF THE SEVERAL VIEWS OF THE DRAWINGS

FIG. 1 illustrates the flow of the blood pressure measurement method of the first embodiment of the present invention.
FIG. 2 illustrates the blood pressure measurement device of the first embodiment of the present invention.
FIG. 3 illustrates the pressure on the wearable pressing unit as a function of time in the first embodiment.
FIG. 4 illustrates a first and second wave signal of the first embodiment.
FIG. 5 illustrates a variation of generating an envelope signal of the first embodiment.
FIG. 6 illustrates a variation of the envelope signal and modified envelope signal in the first embodiment.
FIG. 7 illustrates a variation of the pressure on the wearable pressing unit as a function of time in the first embodiment.
FIG. 8 illustrates the flow of generating an envelope signal in the second embodiment of the present invention.
FIG. 9 illustrates an envelope signal of the second embodiment.
FIG. 10 illustrates an envelope signal and the associated modified envelope signal of the second embodiment.
FIG. 11 illustrates the pressure on the wearable pressing unit as a function of time in the second embodiment.
FIG. 12 illustrates the flow of the blood pressure measurement method of the third embodiment of the present invention.
FIG. 13 illustrates a result of continuous blood pressure measurement according to the blood pressure measurement method of the third embodiment.

### DESCRIPTION OF EMBODIMENTS

The present invention provides a new and improved methods for determining blood pressure there remains a need to produce an improved blood pressure measurement reading that reduces errors and increases increasing an accuracy of measuring actual blood pressure.

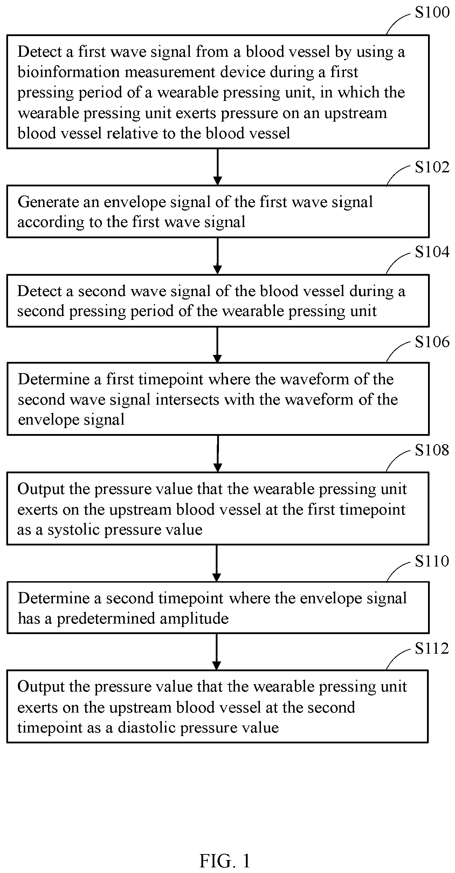

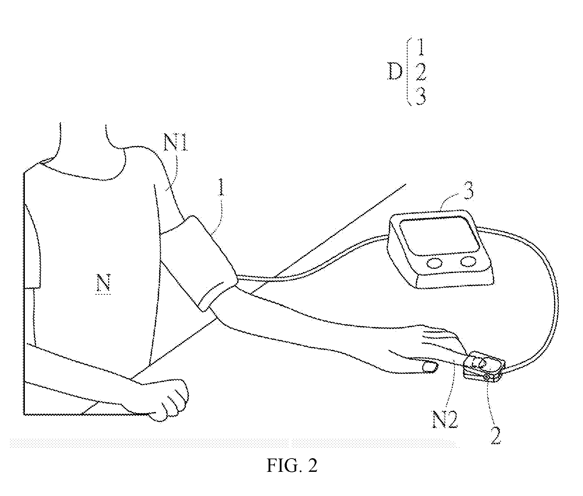

FIG. 1 illustrates an example of a process for obtaining a blood pressure measurement. FIG. 2 depicts an exemplary configuration of a blood pressure measurement device for use with the improved process for measuring blood pressure. The blood pressure measurement device in FIG. 2 includes a wearable pressing unit 1, such as a blood pressure cuff that applies pressure to an arm N1 of the individual N, a bioinformation measurement device 2, and a control unit 3. In this example, the control unit 3 connects to the wearable pressing unit 1 and the bioinformation measurement device 2 to provide one or more signals that execute the blood pressure measurement method as outlined in FIG. 1. In FIG. 1, a first variation of a blood pressure measurement method of includes a step S100: detecting a first wave signal from a blood vessel by using the bioinformation measurement device 2. The wave signal is generally a blood pressure waveform produced by the body during the action of heart pumping blood through the vessel. In the present example, the measurement device 2 detects the first wave signal during a first pressing duration P1 of the wearable pressing unit 1, in which the wearable pressing unit 1 exerts pressure on an upstream blood vessel relative to the blood vessel being measured by the measurement device 2. Specifically, the wearable pressure unit 1 can be a blood pressure cuff that inflates and deflates in a manner controlled by control unit 3; the bioinformation measurement device 2 can be a finger-clip device that includes any sensor that can provide a measurement of pressure (e.g., a pressure sensor). Therefore, the system measures the pressure in the upstream blood vessel in arm N1. In addition, measurement device 2 measures the first wave signal is a blood pressure signal from a vessel in finger N2.

It is noted that the flow charts shown in FIGS. 1, 5, and 8 are intended to detail acts relating to the system and/or method. Each item or box identified in the figures is intended for convenience only. It is contemplated, that the features of the flowchart can combine the acts occurring in the separately identified elements or that the elements can be arranged in a different sequential order, unless otherwise noted.

The methods of the present disclosure can include devices apart from the blood pressure measurement device described above. In some examples, the wearable pressing unit 1 can be any pressing device other than the blood pressure cuff as long as it is able to controllably apply pressure to the upstream blood vessel. In additional variations, the location the wearable pressing unit 1 is not limited to the arm N1. For example, the pressing location may be the wrist N3 of the individual N as long as the bioinformation measurement device 2 detects blood pressure signal from a vessel N2 downstream of the vessel being compressed by the pressing unit 1. In additional variations, the sensor of the finger-clip can be a pressure sensor, an optical sensor, an ultrasound sensor, an electromagnetic sensor, etc., or a combination thereof.

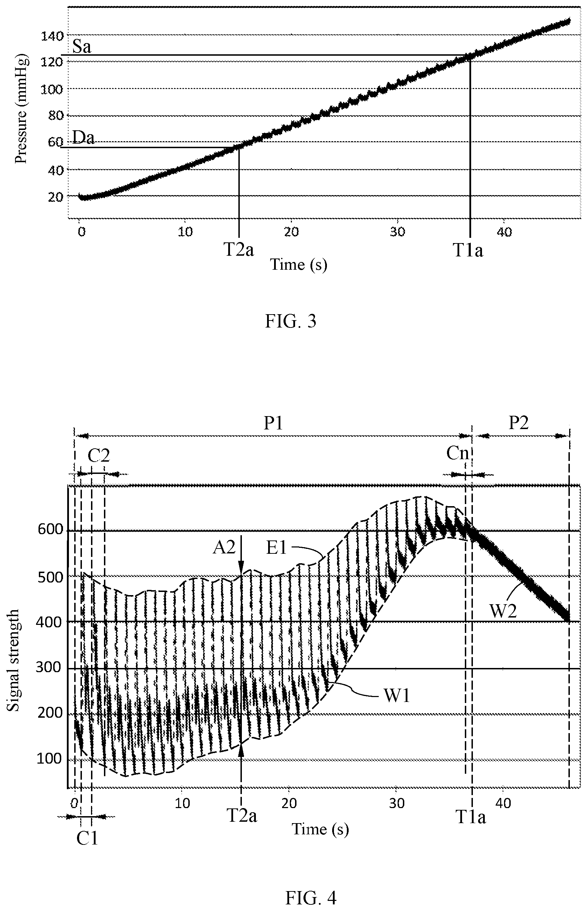

FIG. 4 illustrates a wave signal, where during the first pressing period P1 (i.e., the phase when the pressing unit 1 compresses the artery), the control unit 3 controls inflation of the wearable pressing unit 1. During this first pressing period P1, the bioinformation measurement device 2 detects the first wave signal W1 from the finger N2. FIG. 3 illustrates the pressure, as a function of time, exerted by the wearable pressing unit 1 on the arm N1. During the first pressing period P1, the pressure exerted by the wearable pressing unit 1 on the upstream vessel of the arm N1 increases linearly. As a result, blood flow through the upstream blood vessel gradually decreases with time. This causes the blood flow received by the finger N1 at the downstream vessel to also gradually decrease with time, as illustrated by the decreasing amplitude of the first wave signal W1 in FIG. 4

As shown in FIG. 1, the blood pressure measurement method of the first embodiment further includes step S102: generating an envelope signal E1 of the first wave signal W1 according to the first wave signal W1; step S104: detecting a second wave signal W2 of the blood vessel by using the said bioinformation measurement device 2 during a second pressing period P2 of the wearable pressing unit 1; step 106: determining a first timepoint T1a where the waveform of the second wave signal W2 intersects with the waveform of the envelope signal E1; and step 108: outputting the pressure value that the wearable pressing unit 1 exerts on the upstream blood vessel at the first timepoint T1a as a systolic pressure value. FIGS. 2-4 correlate to the steps S102~S108. As shown in FIG. 4, the first wave signal W1 of the first embodiment includes a plurality of periodic waves (C1, C2, ..., Cn). For the purpose of presentation, FIG. 4 only shows C1, C2, and Cn to illustrate the definition of periodic waves in this example. Specifically, each periodic wave in this variation is defined as the signal between two adjacent valleys of the first wave signal W1. Furthermore, generating the envelope signal E1 occurs by connecting adjacent peaks and valleys of all the periodic waves.

In S104 of the first variation, when the pressure exerted by the wearable pressing unit 1 on the upstream blood vessel exceeds a certain level such that the bioinformation measurement device 2 does not detect waveforms, the wearable pressing unit 1 enters the second pressing period P2. The blood pressure signal detected by the bioinformation measurement device 2 during the second pressing period P2 is the second wave signal W2, as illustrated in FIG. 4. Followingly in steps S106 and S108, the moment when the waveform of the envelope signal E1 intersects with the waveform of the second wave signal W2 is defined as the first timepoint T1a. Furthermore, according to the graph of pressure exerted by the pressing unit 1, as a function of time in FIG. 3, a pressure value Sa is obtained at the first timepoint T1a. The pressure value Sa is then set as the systolic pressure value. Specifically, the envelop signal E1 ends as the first pressing period P1 finishes, and the waveform of the second wave signal W2 starts when the second pressing period P2 begins. In S106, the second timepoint T2a is defined as the moment when the extension of the envelope signal E1 intersects with the waveform of the second wave signal W2. It should be noted that in this variation the second pressing period P2 begins immediately after the first pressing period P1 finishes. Therefore, the first wave signal W1 and the second wave signal W2 are detected within one-time pressing duration of the wearable pressing unit 1. However, additional variations are not limit in this manner. Instead, the first wave signal W1 and the second wave signal W2 can be detected in separated pressing durations. Particularly, the major difference between the first pressing period P1 and the second pressing period P2 is that the bioinformation measurement device 2 can detect systolic and diastolic blood pressure waveforms during the first pressing period P1. On the other hand, the bioinformation measurement device 2 cannot detect blood pressure waveforms during the second pressing period P2 because of poor blood flow in the blood vessel due to compression at the upstream blood vessel by the wearable pressing unit 1. The present invention is not limited to whether the wearable pressing unit 2 increases, reduces, or maintains a constant pressure during the second pressing period P2. Furthermore, this embodiment does not limit the order of steps S102 and S104. The envelope signal E1 can be generated after the first wave signal W1 and the second wave signal W2 are both detected.

After step S108, the blood pressure measurement process further includes S110: determining a second timepoint T2a where the envelope signal E1 has a predetermined amplitude A2; and S112:outputting the pressure value that the wearable pressing unit 1 exerts on the upstream blood vessel at the second timepoint T2a as a diastolic pressure value. In this variation, the predetermined amplitude A2 of the envelope signal E1 is 85% of the maximum amplitude A1. That is, the moment when the amplitude of the envelope signal E1 reduces to 85% of the maximum amplitude A1 is defined as the second timepoint T2a in this variation. However, in additional variations, the predetermined amplitude A2 can be 50%~90% of the maximum amplitude. In other variations, the predetermined amplitude A2 can be decided according to the sample group in which the person belongs to. For example, the sample group can be categorized by heart rate, blood pressure waveform, age, gender, body height, body weight, and etc. Followingly, according to the pressure-time curve in FIG. 3, the pressure Da exerted on the arm N1 by the wearable pressing unit 1 at the second timepoint T2a is outputted as the diastolic pressure value.

In this variation, the diastolic and systolic pressure value can be displayed on a monitor of the control unit 3. However, alternative means of displaying or transmitting the diastolic and systolic pressure are within the scope of this disclosure. In some variations, the bioinformation measurement unit 2 may include a small display to show the diastolic and systolic pressure value.

Through the above-mentioned method, the present invention can directly measure systolic pressure according to the first wave signal W1 detected from a vessel of the person under test N. Comparing to conventional blood pressure measurement method, the present invention does not need to calculate systolic pressure from mean blood pressure and thus improves the accuracy of blood pressure measurement.

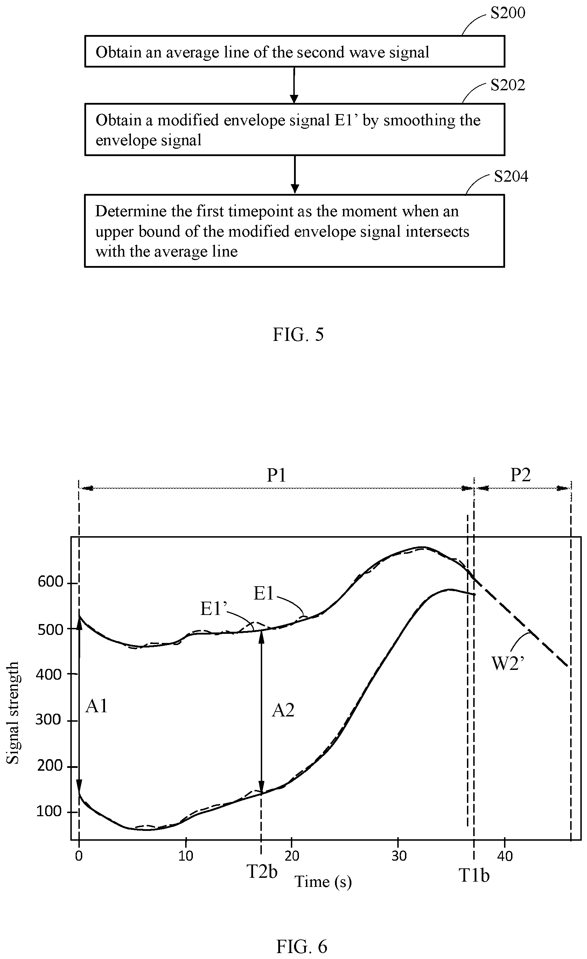

FIGS. 5 and 6 illustrate a variation of the method described herein where S102 can further include S200: obtaining an average line W2' of the second wave signal; S202: obtaining a modified envelope signal E1' by smoothing the envelope signal E1; and S204: determining the first timepoint T1b as the moment when an upper bound of the modified envelope signal E1' intersects with the average line W2'. In S200, the average line W2', as shown in FIG. 6, is a line segment with the signal strength obtained by taking an average of the second wave signal W2. In S202, smoothing the envelope signal E1 can be conducted by fitting the envelope signal E1 with a regressive or interpolative curve. The envelope signal E1 is illustrated by a dashed line, and the modified envelope signal E1' is illustrated by a solid line in FIG. 6. With S202 and S204, the errors in the first wave signal W1 due to motion artifact and respiration variation of the individual N can be reduced. The upstream blood vessel is obstructed when the second wave signal W2 is detected so that the blood flow in the blood vessel in the finger under test is reduced. Therefore, the second wave signal W2 is less affected by motion artifact and respiration variation. Accordingly, in S204, the first timepoint T1b obtained from the modified envelope signal E1' and the average line W2' of the second wave signal W2 is the timepoint of systolic pressure after suppressing errors.

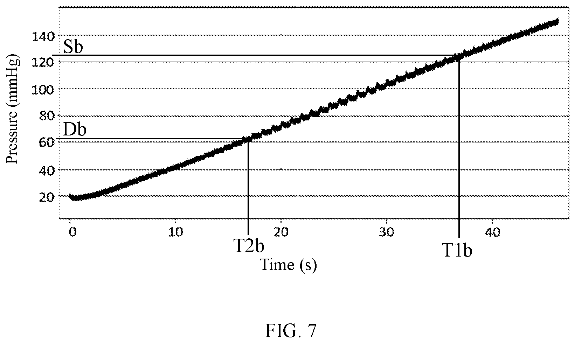

Furthermore, as illustrated in FIGS. 6 and 7, a more accurate systolic value Sb can be obtained from the first time point T1b according to the S200~S204 when S108 is conducted. In addition, in this variation, the second timepoint T2b can be obtained from the timepoint of the predetermined amplitude A2 of the modified envelope signal E1'. Because the modified envelope signal E1' reduces errors caused by motion artifact and respiration variation, the second timepoint T2b obtained from the modified envelope signal E1' is closer to the actual timepoint of diastolic pressure. Therefore, the diastolic pressure value Db obtained from the second timepoint T2b is more accurate.

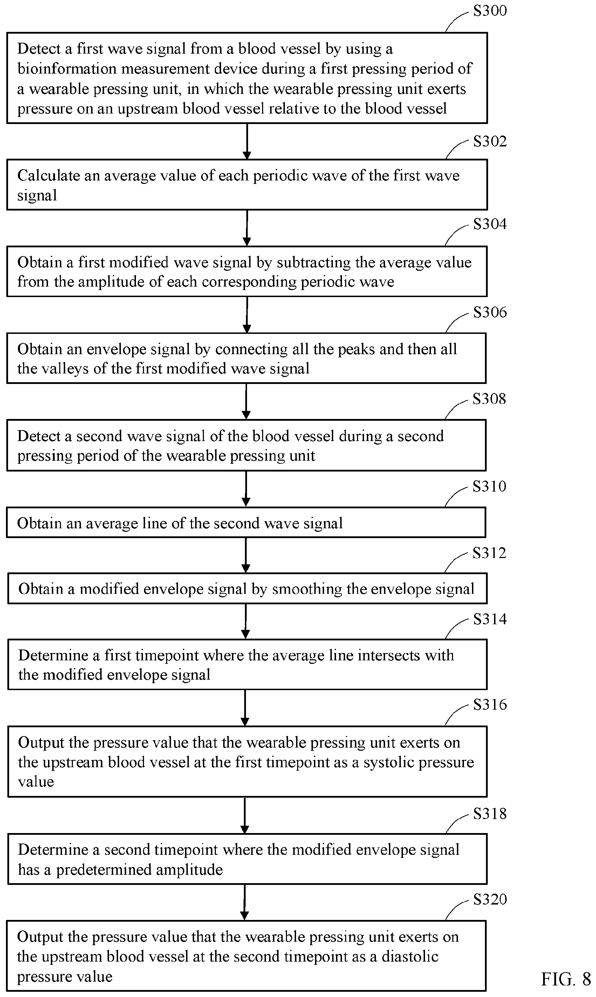

FIGS. 8-11 show another variation of a method for determining blood pressure. In this variation, S102: obtaining an envelope signal of the first wave signal in the variation also includes S302: calculating an average value of each periodic wave of the first wave signal W1; S304: obtaining a first modified wave signal W1' by subtracting the average value from the amplitude of each corresponding periodic wave; and S306: obtaining an envelope signal E2 of the first wave signal W1 by connecting all the peaks and then all the valleys of the first modified wave signal W1'. Specifically, in this embodiment the average value of each periodic wave (C1, C2, ..., Cn) of the first wave signal W1 in FIG. 4 is first calculated. Then a first modified signal W1' is obtained by subtracting the calculated average value from the amplitude of each corresponding periodic wave (C1, C2, ..., Cn). For example, the average value of the periodic wave C1 is subtracted from the amplitude of the periodic wave C1, the average value of the periodic wave C2 is subtracted from the amplitude of the periodic wave C2, and so on. Then, the envelope signal E2 of the second embodiment is obtained by connecting all the peaks and then all the valleys of the first modified wave signal W1'.

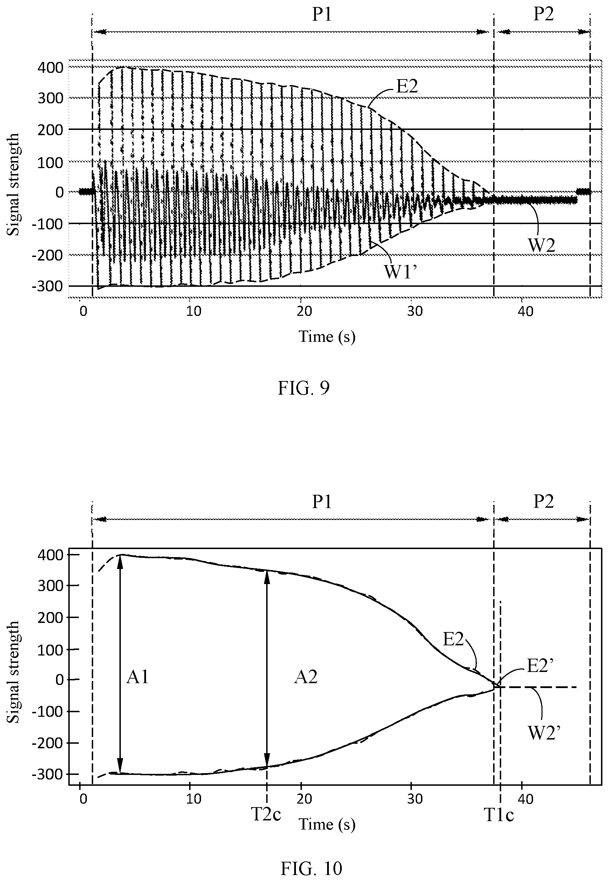

S302 and S304 work as a high pass filter to process the first wave signal W1 in order to remove baseline drift of the first wave signal W1 in FIG. 4. Specifically, as referred by FIG. 2 and FIG. 4, when the wearable pressing unit 1 continuously applies pressure during the pressing period P1, the upstream blood vessel in the arm N1 is compressed above a degree. This causes the blood vessel in finger N2 at the downstream vessel stops acquiring arterial blood readings and that the venous blood at the relative downstream cannot return to the heart through the upstream vessels. As a result, the retained venous blood at the relative downstream causes the baseline of the first wave signal W1 to drift upward, as illustrated by the first wave signal W1 beyond 20 sec in FIG. 4. After the second pressing period P2 begins, water in the venous blood at the relative downstream gradually diffuses out of the vessel. Therefore, the baseline of the second wave signal W2 gradually drifts downward. In addition, low-frequency fluctuations such as respiration variations, vessel motion, and fluid drift in tissue may cause baseline drift of the first wave signal W1. Through S302 and 304 of this variation, the first modified wave signal W1' can be obtained by filtering out baseline drift, as illustrated in FIG. 9. Then, the envelope signal E2 of the second embodiment is obtained by connecting all the peaks and then all the valleys of the first modified wave signal W1'.

Furthermore, the variation detailed in FIG. 5 can be conducted after obtaining the envelope signal E2 . As illustrated in FIG. 8 and FIG. 10, a first timepoint T1c can be obtained by conducting curve-fitting of the first modified wave signal W1. Specifically, in S310 an average line W2' is obtained by calculating an average value of the second wave signal W2. Followingly, in S312 a modified envelope signal E2' is obtained by smoothing the envelope signal E2. Then, the S314 determines a first timepoint T1c where the average line W2' intersects with the modified envelope signal E2'.

In the variations, S314-310 are similar to S106-S112. The modified envelope signal E2' in the second variation replaces the envelope signal E1 of the first embodiment for determining the first timepoint T1c and a second timepoint T2c. In the variation, the first modified signal W1' reduces errors caused by low-frequency fluctuations, and the modified envelope signal E2' further reduces the noise of the first modified signal W1' that is subjected to motion artifact and respiration variations. The noise of the average line W2' due to motion artifact and respiration variations is negligible. Therefore, the first timepoint T1c that is obtained at the intersection of the modified envelope signal E2' and the average line W2' is closer to the actual timepoint of systolic pressure.

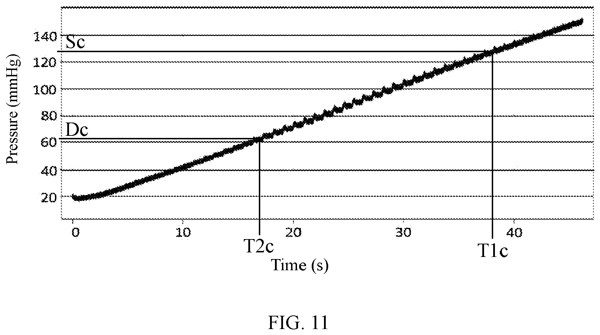

Furthermore, in S318 the obtained second timepoint T2c that is at a predetermined amplitude A2 of the modified envelope signal E2' is closer to the actual timepoint of diastolic pressure because errors due to motion artifact, respiration variations, and signal drift are reduced. As illustrated in FIG. 11, in the second variation the pressure values Sc and Dc exerted by the wearable pressing unit 1 at the first timepoint T1c and the second timepoint T2c, respectively on the upstream blood vessel are closer to the actual systolic pressure and diastolic pressure of the person under test. In view of this, the steps S302~S306 of the second embodiment increases the accuracy of blood pressure measurement by reducing the errors caused by the drift of the first wave signal.

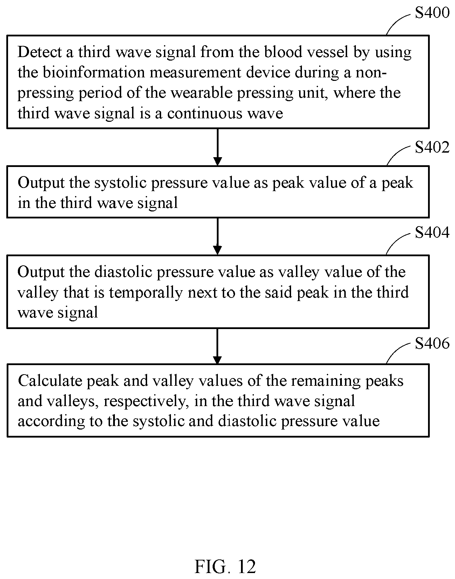

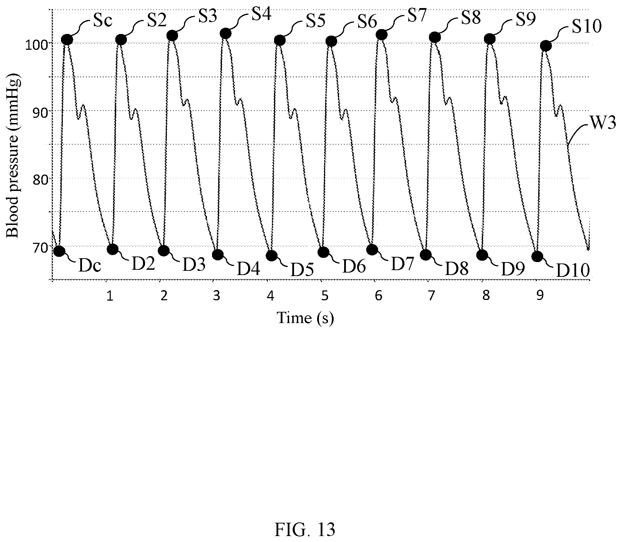

FIGS. 12 and 13 represent an additional variation of a blood pressure measurement method. This variation uses the blood pressure measurement device D described above and can be applied after the first embodiment. The blood pressure measurement method of this variation includes S400: detecting a third wave signal from the blood vessel by using the bioinformation measurement device during a non-pressing period of the wearable pressing unit, where the third wave signal is a continuous wave; S402: outputting the systolic pressure value Sc as peak value of a peak in the third wave signal; S404: outputting the diastolic pressure value Dc as valley value of the valley that is temporally next to the said peak in the third wave signal; and S406: calculating peak and valley values of the remaining peaks and valleys, respectively, in the third wave signal according to the systolic and diastolic pressure value.

FIG. 2 and FIG. 13 also specifically show S400, in which the third wave signal W3 is detected by the bioinformation measurement device 2 on the finger N2 when the wearable pressing unit 1 is not pressing the upstream blood vessel. In S402, the present embodiment outputs the systolic pressure value Sc, that is obtained by the second embodiment, as the peak value of the first peak in the third wave signal W3. In S404, the present variation outputs the diastolic pressure value Dc, that is obtained by the second variation, as the valley value of the first valley in the third wave signal W3. Followingly, in S406, the present variation linearly scales the vertical axis of the third wave signal W3 according to the systolic pressure value Sc and the diastolic pressure value Dc. Therefore, the values of the remaining peaks and valleys in the third wave signal W3 can be calculated. Because the systolic pressure value Sc and the diastolic pressure value Dc are directly measured through signal processing of blood pressure waveform, the remaining systolic and diastolic pressure values of a continuous blood pressure waveform are more accurate if calculated based on the systolic pressure value Sc and the diastolic pressure value Dc. It should be noted that in S402 of other embodiments the systolic pressure value Sc may be outputted as the peak value of any peak in the third wave signal W3. In S404 of other embodiments the diastolic pressure value Dc may be outputted as the valley value of any valley in the third wave signal W3. The present invention is not limited to the embodiment illustrated in FIG. 13.

Well-known structures, materials, or operations are not shown or described in detail to avoid obscuring aspects of the described devices. As those skilled in the art would realize, the described embodiments may be modified in various different ways, but the scope is defined by the claims. It should be noted that, without conflict, in the embodiment of the present invention and examples of features can be combined with each other. Therefore, it should be appreciated that the embodiments described herein are not intended to be exhaustive of all possible embodiments in accordance with the present disclosure, and that additional embodiments may be conceived based on the subject matter disclosed herein.

## Claims

1. A system for blood pressure measurement of an individual, the system comprising:
a controller (3) coupled to a pressing unit (1) and to a bioinformation measurement device (2);
wherein the controller (3) is configured to incrementally increase a pressure applied by the pressing unit (1) on a body part of an individual during a first pressing period (P1) and a second pressing period (P2) to compress the body part such that the pressing unit (1) exerts the pressure on an upstream blood vessel relative to a downstream blood vessel measured by the bioinformation measurement device (2);
wherein the bioinformation measurement device (2) is configured to produce a first wave signal (W1) that is representative of blood pressure activity from the downstream blood vessel during the first pressing period (P1) and transmitting the first wave signal (W1) to the controller (3);
wherein the controller (3) generates an envelope signal (E1) using the first wave signal (W1);
wherein the controller (3) is configured to produce a second wave signal (W2) that is representative of blood pressure activity from the downstream blood vessel during the second pressing period (P2) and transmitting the second wave signal (W2) to the controller (3), wherein the second pressing period (P2) begins when the bioinformation measurement device (2) fails to detect the blood pressure activity from the downstream blood vessel;
wherein the controller (3) determines a first timepoint (T1a) by determining where a waveform of the second wave signal (W2) intersects with a waveform of the envelope signal (E1), wherein the controller (3) determines a systolic pressure value (Sa) by determining a first pressure value that the pressing unit (1) exerts on the upstream blood vessel at the first timepoint (T1a); and
wherein the controller (3) determines a second timepoint (T2a) by determining where the envelope signal (E1) has a predetermined amplitude (A2), wherein the controller (3) determines a diastolic pressure value (Da) by determining a second pressure value that the pressing unit (1) exerts on the upstream blood vessel at the second timepoint (T2a).

2. The system of claim 1, wherein the first wave signal (W1) has a plurality of periodic waves (C1, C2, ..., Cn), and wherein the controller generates the envelope signal (E2) using the first wave signal (W1) comprises:
calculating an average value of each periodic wave of the first wave signal (W1) (S302);
obtaining a first modified wave signal (W1') by subtracting the average value from the predetermined amplitude of each corresponding periodic wave (C1, C2, ..., Cn) (S304); and
obtaining the envelope signal (E2) by connecting a plurality of peaks of the first modified wave signal (W1') and a plurality of valleys of the first modified wave signal (W1') (S306).

3. The system of claim 1, wherein the first wave signal (W1) has a plurality of periodic waves (C1, C2, ..., Cn), wherein the controller (3) generates the envelope signal (E1) by connecting peaks of each periodic wave (C1, C2, ..., Cn) and valleys of each periodic wave (C1, C2, ..., Cn) (S306).

4. The system of claim 1 where the controller (3) is further configured to:
detect a third wave signal (W3) from the upstream blood vessel by using the bioinformation measurement device (2) during a non-pressing period of the pressing unit (1), where the third wave signal (W3) comprises a continuous wave;
output the systolic pressure value (Sa) as an initial peak value of a peak in the third wave signal (W3);
output the diastolic pressure value (Da) as an initial valley value of a valley in the third wave signal (W3) that is temporally adjacent to the peak in the third wave signal (W3); and
calculate a plurality of additional peak values and a plurality of additional valley value of a plurality of remaining peaks and valleys, respectively, in the third wave signal (W3) according to the systolic pressure value (Sa) and the diastolic pressure value (Da) (S406).

5. The system of claim 1, wherein the controller (3) is configured to determine the first timepoint (T1b) by:
obtaining an average line (W2') of the second wave signal (W2);
obtaining a modified envelope signal (E1') by smoothing the envelope signal (E1); and
determine the first timepoint (T1b) as a time when an upper bound of the modified envelope signal (E1') intersects with the average line (W2').

6. The system of claim 1, wherein the predetermined amplitude (A2) of the envelope signal (E1) is between 50% and 90% of a maximum amplitude (A1) of the envelope signal (E1).

7. The system of claim 1, wherein the pressing unit (1) is configured to fit on an arm or a wrist, wherein the controller (3) is configured to cause the pressing unit (1) to apply pressure during the first pressing period (P1) and the second pressing period (P2), and wherein the bioinformation measurement device (2) is configured to detect the first wave signal (W1) and the second wave signal (W2) from a finger.

8. A blood pressure measurement method comprising:
detecting a first pressure wave signal (W1) from a blood vessel by using a bioinformation measurement device (2) during a first pressing period (P1) of a wearable pressing unit (1), in which the wearable pressing unit (1) exerts pressure on an upstream blood vessel relative to the blood vessel;
generating an envelope signal (E1) of the first pressure wave signal (W1) according to the first pressure wave signal (W1);
detecting a second pressure wave signal (W2) of the blood vessel by using the bioinformation measurement device (2) during a second pressing period (P2) of the wearable pressing unit (1);
determining a first timepoint (T1a) where a waveform of the second pressure wave signal (W2) intersects with a waveform of the envelope signal (E1);
outputting a pressure value that the wearable pressing unit (1) exerts on the upstream blood vessel at the first timepoint (T1a) as a systolic pressure value (Sa);
determining a second timepoint (T2a) where the envelope signal (E1) has a predetermined amplitude (A2); and
outputting the pressure value that the wearable pressing unit (1) exerts on the upstream blood vessel at the second timepoint (T2a) as a diastolic pressure value (Da).

9. The method of claim 8, wherein the first pressure wave signal (W1) has a plurality of periodic waves (C1, C2, ..., Cn) and generating the envelope signal (E2) further comprises:
calculating an average value of each periodic wave (C1, C2, ..., Cn) of the first pressure wave signal (W1);
obtaining a first modified wave signal (W1') by subtracting the average value from an amplitude of each corresponding periodic wave (C1, C2, ..., Cn); and
obtaining the envelope signal (E2) by connecting all the peaks and then all the valleys of the first modified wave signal (W1').

10. The method of claim 8, wherein the first pressure wave signal (W1) has a plurality of periodic waves (C1, C2, ..., Cn) and generating the envelope signal (E1) further comprises obtaining the envelope signal (E1) by connecting peaks of each periodic wave (C1, C2, ..., Cn) and valleys of each periodic wave (C1, C2, ..., Cn).

11. The method of claim 8, further comprising:
detecting a third wave signal (W3) from the blood vessel by using the bioinformation measurement device (2) during a non-pressing period of the wearable pressing unit (1), where the third wave signal (W3) is a continuous wave;
outputting the systolic pressure value (Sa) as a peak value of a peak in the third wave signal (W3);
outputting the diastolic pressure value (Da) as a valley value of the valley that is temporally adjacent to the peak in the third wave signal (W3); and
calculating peak and valley values of the remaining peaks and valleys, respectively, in the third wave signal (W3) according to the systolic pressure value (Sa) and the diastolic pressure value (Da).

12. The method of claim 8, wherein determining the first timepoint (T1b) further comprises:
obtaining an average line (W2') of the second wave signal (W2);
obtaining a modified envelope signal (E1') by smoothing the envelope signal (E1); and
determining the first timepoint (T1b) as a time when an upper bound of the modified envelope signal (E1') intersects with the average line (W2').

13. The method of claim 8, wherein the predetermined amplitude (A2) of the envelope signal (E1) is between 50% and 90% of a maximum amplitude (A1) of the envelope signal (E1).

14. The method of claim 8, wherein the wearable pressing unit (1) applies pressure on an arm or a wrist during the first (P1) and the second pressing period (P2), and wherein the bioinformation measurement device (2) detects the first pressure wave signal (W1) and the second pressure wave signal (W2) from a finger.

## Patentansprüche

1. System zur Blutdruckmessung bei einer Person, wobei das System umfasst:
eine Steuereinheit (3), die mit einer Druckeinheit (1) und mit einer Bioinformations-Messvorrichtung (2) gekoppelt ist;
wobei die Steuereinheit (3) dazu ausgelegt ist, einen von der Druckeinheit (1) auf einen Körperteil einer Person ausgeübten Druck während einer ersten Druckperiode (P1) und einer zweiten Druckperiode (P2) schrittweise zu erhöhen, um den Körperteil so zu komprimieren, dass die Druckeinheit (1) den Druck auf ein stromaufwärts gelegenes Blutgefäß relativ zu einem stromabwärts gelegenen Blutgefäß ausübt, das von der Bioinformations-Messvorrichtung (2) gemessen wird;
wobei die Bioinformations-Messvorrichtung (2) dazu ausgelegt ist, während der ersten Druckperiode (P1) ein erstes Wellensignal (W1) zu erzeugen, das für eine Blutdruckaktivität aus dem stromabwärts gelegenen Blutgefäß repräsentativ ist, und das erste Wellensignal (W1) an die Steuereinheit (3) zu übertragen;
wobei die Steuereinheit (3) unter Verwendung des ersten Wellensignals (W1) ein Hüllkurvensignal (E1) erzeugt;
wobei die Steuereinheit (3) dazu ausgelegt ist, während der zweiten Druckperiode (P2) ein zweites Wellensignal (W2) zu erzeugen, das für eine Blutdruckaktivität aus dem stromabwärts gelegenen Blutgefäß repräsentativ ist, und das zweite Wellensignal (W2) an die Steuereinheit (3) zu übertragen, wobei die zweite Druckperiode (P2) beginnt, wenn die Bioinformations-Messvorrichtung (2) die Blutdruckaktivität aus dem stromabwärts gelegenen Blutgefäß nicht mehr detektiert;
wobei die Steuereinheit (3) einen ersten Zeitpunkt (T1a) bestimmt, indem sie bestimmt, wo sich eine Wellenform des zweiten Wellensignals (W2) mit einer Wellenform des Hüllkurvensignals (E1) schneidet, wobei die Steuereinheit (3) einen systolischen Druckwert (Sa) bestimmt, indem sie einen ersten Druckwert bestimmt, den die Druckeinheit (1) zum ersten Zeitpunkt (T1a) auf das stromaufwärts gelegene Blutgefäß ausübt; und
wobei die Steuereinheit (3) einen zweiten Zeitpunkt (T2a) bestimmt, indem sie bestimmt, wo das Hüllkurvensignal (E1) eine vorbestimmte Amplitude (A2) aufweist, wobei die Steuereinheit (3) einen diastolischen Druckwert (Da) bestimmt, indem sie einen zweiten Druckwert bestimmt, den die Druckeinheit (1) zum zweiten Zeitpunkt (T2a) auf das stromaufwärts gelegene Blutgefäß ausübt.

2. System nach Anspruch 1, wobei das erste Wellensignal (W1) eine Mehrzahl periodischer Wellen (C1, C2, ... , Cn) aufweist, und wobei das Erzeugen des Hüllkurvensignals (E2) durch die Steuereinheit unter Verwendung des ersten Wellensignals (W1) umfasst:
Berechnen eines Mittelwerts jeder periodischen Welle des ersten Wellensignals (W1) (S302); Erhalten eines ersten modifizierten Wellensignals (W1'), indem der Mittelwert von der vorbestimmten Amplitude jeder entsprechenden periodischen Welle (C1, C2, ... , Cn) subtrahiert wird (S304); und
Erhalten des Hüllkurvensignals (E2), indem eine Mehrzahl von Spitzen des ersten modifizierten Wellensignals (W1') und eine Mehrzahl von Tälern des ersten modifizierten Wellensignals (W1') miteinander verbunden werden (S306).

3. System nach Anspruch 1, wobei das erste Wellensignal (W1) eine Mehrzahl periodischer Wellen (C1, C2, ... , Cn) aufweist, wobei die Steuereinheit (3) das Hüllkurvensignal (E1) erzeugt, indem Spitzen jeder periodischen Welle (C1, C2, ... , Cn) und Täler jeder periodischen Welle (C1, C2, ... , Cn) miteinander verbunden werden (S306).

4. System nach Anspruch 1, wobei die Steuereinheit (3) ferner dazu ausgelegt ist:
während einer druckfreien Periode der Druckeinheit (1) unter Verwendung der Bioinformations-Messvorrichtung (2) ein drittes Wellensignal (W3) aus dem stromaufwärts gelegenen Blutgefäß zu detektieren, wobei das dritte Wellensignal (W3) eine kontinuierliche Welle umfasst;
den systolischen Druckwert (Sa) als einen anfänglichen Spitzenwert einer Spitze in dem dritten Wellensignal (W3) auszugeben;
den diastolischen Druckwert (Da) als einen anfänglichen Talwert eines Tals in dem dritten Wellensignal (W3) auszugeben, das zeitlich an die Spitze in dem dritten Wellensignal (W3) angrenzt; und
eine Mehrzahl zusätzlicher Spitzenwerte und eine Mehrzahl zusätzlicher Talwerte der Mehrzahl verbleibender Spitzen bzw. Täler in dem dritten Wellensignal (W3) gemäß dem systolischen Druckwert (Sa) und dem diastolischen Druckwert (Da) zu berechnen (S406).

5. System nach Anspruch 1, wobei die Steuereinheit (3) dazu ausgelegt ist, den ersten Zeitpunkt (T1b) zu bestimmen durch:
Erhalten einer Mittellinie (W2') des zweiten Wellensignals (W2);
Erhalten eines modifizierten Hüllkurvensignals (E1'), indem das Hüllkurvensignal (E1) geglättet wird; und Bestimmen des ersten Zeitpunkts (T1b) als einen Zeitpunkt, zu dem sich eine Obergrenze des modifizierten Hüllkurvensignals (E1') mit der Mittellinie (W2') schneidet.

6. System nach Anspruch 1, wobei die vorbestimmte Amplitude (A2) des Hüllkurvensignals (E1) zwischen 50 % und 90 % einer maximalen Amplitude (A1) des Hüllkurvensignals (E1) liegt.

7. System nach Anspruch 1, wobei die Druckeinheit (1) dazu ausgelegt ist, an einem Arm oder einem Handgelenk angebracht zu werden, wobei die Steuereinheit (3) dazu ausgelegt ist, die Druckeinheit (1) zu veranlassen, während der ersten Druckperiode (P1) und der zweiten Druckperiode (P2) Druck auszuüben, und
wobei die Bioinformations-Messvorrichtung (2) dazu ausgelegt ist, das erste Wellensignal (W1) und das zweite Wellensignal (W2) von einem Finger zu detektieren.

8. Blutdruckmessverfahren, umfassend:
Detektieren eines ersten Druckwellensignals (W1) aus einem Blutgefäß unter Verwendung einer Bioinformations-Messvorrichtung (2) während einer ersten Druckperiode (P1) einer tragbaren Druckeinheit (1), in der die tragbare Druckeinheit (1) Druck auf ein stromaufwärts gelegenes Blutgefäß relativ zu dem Blutgefäß ausübt;
Erzeugen eines Hüllkurvensignals (E1) des ersten Druckwellensignals (W1) gemäß dem ersten Druckwellensignal (W1);
Detektieren eines zweiten Druckwellensignals (W2) des Blutgefäßes unter Verwendung der Bioinformations-Messvorrichtung (2) während einer zweiten Druckperiode (P2) der tragbaren Druckeinheit (1);
Bestimmen eines ersten Zeitpunkts (T1a), an dem sich eine Wellenform des zweiten Druckwellensignals (W2) mit einer Wellenform des Hüllkurvensignals (E1) schneidet;
Ausgeben eines Druckwerts, den die tragbare Druckeinheit (1) zum ersten Zeitpunkt (T1a) auf das stromaufwärts gelegene Blutgefäß ausübt, als systolischen Druckwert (Sa); Bestimmen eines zweiten Zeitpunkts (T2a),
an dem das Hüllkurvensignal (E1) eine vorbestimmte Amplitude (A2) aufweist; und
Ausgeben des Druckwerts, den die tragbare Druckeinheit (1) zum zweiten Zeitpunkt (T2a) auf das stromaufwärts gelegene Blutgefäß ausübt, als diastolischen Druckwert (Da).

9. Verfahren nach Anspruch 8, wobei das erste Druckwellensignal (W1) eine Mehrzahl periodischer Wellen (C1, C2, ... , Cn) aufweist und das Erzeugen des Hüllkurvensignals (E2) ferner umfasst:
Berechnen eines Mittelwerts jeder periodischen Welle (C1, C2, ... , Cn) des ersten Druckwellensignals (W1);
Erhalten eines ersten modifizierten Wellensignals (W1'), indem der Mittelwert von einer Amplitude jeder entsprechenden periodischen Welle (C1, C2, ... , Cn) subtrahiert wird; und
Erhalten des Hüllkurvensignals (E2), indem zunächst alle Spitzen und danach alle Täler des ersten modifizierten Wellensignals (W1') miteinander verbunden werden.

10. Verfahren nach Anspruch 8, wobei das erste Druckwellensignal (W1) eine Mehrzahl periodischer Wellen (C1, C2, ... , Cn) aufweist und das Erzeugen des Hüllkurvensignals (E1) ferner umfasst, das Hüllkurvensignal (E1) zu erhalten, indem Spitzen jeder periodischen Welle (C1, C2, ... , Cn) und Täler jeder periodischen Welle (C1, C2, ... , Cn) miteinander verbunden werden.

11. Verfahren nach Anspruch 8, ferner umfassend:
Detektieren eines dritten Wellensignals (W3) aus dem Blutgefäß unter Verwendung der Bioinformations-Messvorrichtung (2) während einer druckfreien Periode der tragbaren Druckeinheit (1), wobei das dritte Wellensignal (W3) eine kontinuierliche Welle ist;
Ausgeben des systolischen Druckwerts (Sa) als Spitzenwert einer Spitze in dem dritten Wellensignal (W3);
Ausgeben des diastolischen Druckwerts (Da) als Talwert des Tals, das zeitlich an die Spitze in dem dritten Wellensignal (W3) angrenzt; und
Berechnen von Spitzen- und Talwerten der verbleibenden Spitzen bzw. Täler in dem dritten Wellensignal (W3) gemäß dem systolischen Druckwert (Sa) und dem diastolischen Druckwert (Da).

12. Verfahren nach Anspruch 8, wobei das Bestimmen des ersten Zeitpunkts (T1b) ferner umfasst: Erhalten einer Mittellinie (W2') des zweiten Wellensignals (W2);
Erhalten eines modifizierten Hüllkurvensignals (E1'), indem das Hüllkurvensignal (E1) geglättet wird; und Bestimmen des ersten Zeitpunkts (T1b) als einen Zeitpunkt, zu dem sich eine Obergrenze des modifizierten Hüllkurvensignals (E1') mit der Mittellinie (W2') schneidet.

13. Verfahren nach Anspruch 8, wobei die vorbestimmte Amplitude (A2) des Hüllkurvensignals (E1) zwischen 50 % und 90 % einer maximalen Amplitude (A1) des Hüllkurvensignals (E1) liegt.

14. Verfahren nach Anspruch 8, wobei die tragbare Druckeinheit (1) während der ersten (P1) und der zweiten Druckperiode (P2) Druck auf einen Arm oder ein Handgelenk ausübt, und wobei die Bioinformations-Messvorrichtung (2) das erste Druckwellensignal (W1) und das zweite Druckwellensignal (W2) von einem Finger detektiert.

## Revendications

1. Système de mesure de la pression artérielle d'un individu, le système comprenant :
un contrôleur (3) couplé à une unité de pression (1) et à un dispositif de mesure d'informations biologiques (2) ;
dans lequel le contrôleur (3) est configuré pour augmenter progressivement une pression appliquée par l'unité de pression (1) sur une partie du corps d'un individu pendant une première période de pression (P1) et une deuxième période de pression (P2) afin de comprimer la partie du corps de telle sorte que l'unité de pression (1) exerce la pression sur un vaisseau sanguin amont par rapport à un vaisseau sanguin aval mesuré par le dispositif de mesure d'informations biologiques (2) ;
dans lequel le dispositif de mesure d'informations biologiques (2) est configuré pour produire un premier signal d'onde (W1) représentatif d'une activité de pression artérielle provenant du vaisseau sanguin aval pendant la première période de pression (P1) et transmettre le premier signal d'onde (W1) au contrôleur (3) ;
dans lequel le contrôleur (3) génère un signal d'enveloppe (E1) en utilisant le premier signal d'onde (W1) ;
dans lequel le contrôleur (3) est configuré pour produire un deuxième signal d'onde (W2) représentatif d'une activité de pression artérielle provenant du vaisseau sanguin aval pendant la deuxième période de pression (P2) et transmettre le deuxième signal d'onde (W2) au contrôleur (3), la deuxième période de pression (P2) commençant lorsque le dispositif de mesure d'informations biologiques (2) ne parvient pas à détecter l'activité de pression artérielle provenant du vaisseau sanguin aval ;
dans lequel le contrôleur (3) détermine un premier instant (T1a) en déterminant où une forme d'onde du deuxième signal d'onde (W2) intersecte une forme d'onde du signal d'enveloppe (E1), le contrôleur (3) déterminant une valeur de pression systolique (Sa) en déterminant une première valeur de pression que l'unité de pression (1) exerce sur le vaisseau sanguin amont au premier instant (T1a) ; et
dans lequel le contrôleur (3) détermine un deuxième instant (T2a) en déterminant où le signal d'enveloppe (E1) a une amplitude prédéterminée (A2), le contrôleur (3) déterminant une valeur de pression diastolique (Da) en déterminant une deuxième valeur de pression que l'unité de pression (1) exerce sur le vaisseau sanguin amont au deuxième instant (T2a).

2. Système selon la revendication 1, dans lequel le premier signal d'onde (W1) comporte une pluralité d'ondes périodiques (C1, C2, ... , Cn), et dans lequel le contrôleur génère le signal d'enveloppe (E2) en utilisant le premier signal d'onde (W1), ce qui comprend :
le calcul d'une valeur moyenne de chaque onde périodique du premier signal d'onde (W1) (S302) ; l'obtention d'un premier signal d'onde modifié (W1') en soustrayant la valeur moyenne de l'amplitude prédéterminée de chaque onde périodique correspondante (C1, C2, ... , Cn) (S304) ; et
l'obtention du signal d'enveloppe (E2) en reliant une pluralité de pics du premier signal d'onde modifié (W1') et une pluralité de vallées du premier signal d'onde modifié (W1') (S306).

3. Système selon la revendication 1, dans lequel le premier signal d'onde (W1) comporte une pluralité d'ondes périodiques (C1, C2, ... , Cn), dans lequel le contrôleur (3) génère le signal d'enveloppe (E1) en reliant les pics de chaque onde périodique (C1, C2, ... , Cn) et les vallées de chaque onde périodique (C1, C2, ... , Cn) (S306).

4. Système selon la revendication 1, dans lequel le contrôleur (3) est en outre configuré pour :
détecter un troisième signal d'onde (W3) provenant du vaisseau sanguin amont en utilisant le dispositif de mesure d'informations biologiques (2) pendant une période sans pression de l'unité de pression (1), le troisième signal d'onde (W3) comprenant une onde continue ;
sortir la valeur de pression systolique (Sa) en tant que valeur de pic initiale d'un pic dans le troisième signal d'onde (W3) ;
sortir la valeur de pression diastolique (Da) en tant que valeur de vallée initiale d'une vallée dans le troisième signal d'onde (W3) qui est temporellement adjacente au pic dans le troisième signal d'onde (W3) ; et
calculer une pluralité de valeurs de pic supplémentaires et une pluralité de valeurs de vallée supplémentaires de la pluralité de pics et de vallées restants, respectivement, dans le troisième signal d'onde (W3) en fonction de la valeur de pression systolique (Sa) et de la valeur de pression diastolique (Da) (S406).

5. Système selon la revendication 1, dans lequel le contrôleur (3) est configuré pour déterminer le premier instant (T1b) par :
l'obtention d'une ligne moyenne (W2') du deuxième signal d'onde (W2) ;
l'obtention d'un signal d'enveloppe modifié (E1') par lissage du signal d'enveloppe (E1) ; et la détermination du premier instant (T1b) comme étant un instant auquel une borne supérieure du signal d'enveloppe modifié (E1') intersecte la ligne moyenne (W2').

6. Système selon la revendication 1, dans lequel l'amplitude prédéterminée (A2) du signal d'enveloppe (E1) est comprise entre 50 % et 90 % d'une amplitude maximale (A1) du signal d'enveloppe (E1).

7. Système selon la revendication 1, dans lequel l'unité de pression (1) est configurée pour être placée sur un bras ou un poignet, dans lequel le contrôleur (3) est configuré pour amener l'unité de pression (1) à appliquer une pression pendant la première période de pression (P1) et la deuxième période de pression (P2), et
dans lequel le dispositif de mesure d'informations biologiques (2) est configuré pour détecter le premier signal d'onde (W1) et le deuxième signal d'onde (W2) à partir d'un doigt.

8. Procédé de mesure de la pression artérielle comprenant :
la détection d'un premier signal d'onde de pression (W1) provenant d'un vaisseau sanguin en utilisant un dispositif de mesure d'informations biologiques (2) pendant une première période de pression (P1) d'une unité de pression portable (1), dans laquelle l'unité de pression portable (1) exerce une pression sur un vaisseau sanguin amont par rapport au vaisseau sanguin ;
la génération d'un signal d'enveloppe (E1) du premier signal d'onde de pression (W1) selon le premier signal d'onde de pression (W1) ;
la détection d'un deuxième signal d'onde de pression (W2) du vaisseau sanguin en utilisant le dispositif de mesure d'informations biologiques (2) pendant une deuxième période de pression (P2) de l'unité de pression portable (1) ;
la détermination d'un premier instant (T1a) où une forme d'onde du deuxième signal d'onde de pression (W2) intersecte une forme d'onde du signal d'enveloppe (E1) ;
la sortie d'une valeur de pression que l'unité de pression portable (1) exerce sur le vaisseau sanguin amont au premier instant (T1a) comme valeur de pression systolique (Sa) ; la détermination d'un deuxième instant (T2a)
où le signal d'enveloppe (E1) a une amplitude prédéterminée (A2) ; et
la sortie de la valeur de pression que l'unité de pression portable (1) exerce sur le vaisseau sanguin amont au deuxième instant (T2a) comme valeur de pression diastolique (Da).

9. Procédé selon la revendication 8, dans lequel le premier signal d'onde de pression (W1) comporte une pluralité d'ondes périodiques (C1, C2, ... , Cn) et la génération du signal d'enveloppe (E2) comprend en outre :
le calcul d'une valeur moyenne de chaque onde périodique (C1, C2, ... , Cn) du premier signal d'onde de pression (W1) ;
l'obtention d'un premier signal d'onde modifié (W1') en soustrayant la valeur moyenne d'une amplitude de chaque onde périodique correspondante (C1, C2, ... , Cn) ; et
l'obtention du signal d'enveloppe (E2) en reliant d'abord tous les pics puis toutes les vallées du premier signal d'onde modifié (W1').

10. Procédé selon la revendication 8, dans lequel le premier signal d'onde de pression (W1) comporte une pluralité d'ondes périodiques (C1, C2, ... , Cn) et la génération du signal d'enveloppe (E1) comprend en outre l'obtention du signal d'enveloppe (E1) en reliant les pics de chaque onde périodique (C1, C2, ... , Cn) et les vallées de chaque onde périodique (C1, C2, ... , Cn).

11. Procédé selon la revendication 8, comprenant en outre :
la détection d'un troisième signal d'onde (W3) provenant du vaisseau sanguin en utilisant le dispositif de mesure d'informations biologiques (2) pendant une période sans pression de l'unité de pression portable (1), le troisième signal d'onde (W3) étant une onde continue ;
la sortie de la valeur de pression systolique (Sa) comme valeur de pic d'un pic dans le troisième signal d'onde (W3) ;
la sortie de la valeur de pression diastolique (Da) comme valeur de vallée de la vallée qui est temporellement adjacente au pic dans le troisième signal d'onde (W3) ; et
le calcul des valeurs de pic et de vallée des pics et vallées restants, respectivement, dans le troisième signal d'onde (W3) en fonction de la valeur de pression systolique (Sa) et de la valeur de pression diastolique (Da).

12. Procédé selon la revendication 8, dans lequel la détermination du premier instant (T1b) comprend en outre : l'obtention d'une ligne moyenne (W2') du deuxième signal d'onde (W2) ;
l'obtention d'un signal d'enveloppe modifié (E1') par lissage du signal d'enveloppe (E1) ; et la détermination du premier instant (T1b) comme étant un instant auquel une borne supérieure du signal d'enveloppe modifié (E1') intersecte la ligne moyenne (W2').

13. Procédé selon la revendication 8, dans lequel l'amplitude prédéterminée (A2) du signal d'enveloppe (E1) est comprise entre 50 % et 90 % d'une amplitude maximale (A1) du signal d'enveloppe (E1).

14. Procédé selon la revendication 8, dans lequel l'unité de pression portable (1) applique une pression sur un bras ou un poignet pendant la première (P1) et la deuxième période de pression (P2), et dans lequel le dispositif de mesure d'informations biologiques (2) détecte le premier signal d'onde de pression (W1) et le deuxième signal d'onde de pression (W2) à partir d'un doigt.
